# EUROPEAN PATENT APPLICATION

(11) **EP 1 862 805 A1**
(43) Date of publication of application: **05.12.2007**
(21) Application number: 06011416.2
(22) Date of filing: 01.06.2006
(51) Int. Cl.: G01N 33/68

(54) **The use of mrp 8/14 levels for discrimination of individuals at risk of acute coronary syndromes**

(71) Applicant: University of Zürich, 8006 Zürich (CH)
(72) Inventor: Maier, Willibald, Dr., 8702 Zollikon Dorf (CH); Altwegg, Lukas, Dr.med., 8006 Zürich (CH); Hersberger, Martin, Dr., 8057 Zürich (CH); Neidhart, Michel, Dr., 8142 Uitikon Waldegg (CH)
(74) Representative: Schwarz, Ralf

(57) **Abstract**

The present invention relates to a method for determining the risk whether an individual showing at least one symptom of an evolving acute coronary syndrome (ACS) is suffering from an acute coronary syndrome comprising the steps of (a) measuring, preferably in vitro, the level of MRP 8/14, wherein (b) if the level of MRP 8/14 is at least increased, then the individual is at risk of suffering from an acute coronary syndrome. The invention further relates to methods for ruling out whether an individual showing at least one symptom of an evolving ACS is suffering from an ACS, for assessing whether an individual showing at least one symptom of an evolving ACS is not at risk of suffering from an ACS and to discriminate if an individual showing at least one symptom of an evolving ACS is at risk to suffer from an ACS and or has no ACS.

## Description

### Field of the invention

The present invention relates to the new use of a marker to diagnose acute coronary syndromes and/or to rule out the likelihood whether an individual showing at least one symptom of an evolving acute coronary syndrome is suffering from an acute coronary syndrome. Further, this invention describes the new use of a diagnostic marker to assist physicians in rapidly discriminating which individuals suffering from chest pain and/or any pertinent clinical symptoms have an acute coronary syndrome before markers of myocardial necrosis are detectable. Due to the new use of this diagnostic marker these individuals will preferentially benefit from rapid and early treatment, leading to improved outcome.

### Background of the invention

Coronary heart diseases (CHD) remain the most common cause of mortality in the western world.

Coronary heart disease, also called coronary artery disease (CAD) and atherosclerotic heart disease is a general term including several coronary heart events which exhibit different risk rates for the patient requiring different treatments and, respectively, different grades of supervision of the individual under examination.

Individuals suffering from a coronary heart disease can be divided into individuals showing no clinical symptoms and those, which present with chest pain and other symptoms such as breathlessness. The latter group can be divided into individuals having stable angina pectoris (SAP) and those with acute coronary syndromes (ACS). ACS patients can show unstable angina pectoris (UAP) or myocardial infarction (MI).

Intravascular and intraluminal and processes interfering with myocardial blood supply are associated with the wide clinical spectrum of CHD. Acute rupture of an atherosclerotic plaque is a severe complication of CAD since intra-coronary thrombus formation with the risk of total coronary occlusion and major clinical events such as ACS including MI and death may occur (Fuster et al., 1999). Recently, inflammation has been suggested as a possible cause of plaque rupture, supported by the observation that inflammatory markers are independent predictors of cardiovascular events in patients with no evidence of cardiovascular disease (Ridker et al., 2000; Cesari et al., 2003). Furthermore levels of various inflammatory markers are elevated in acute coronary syndromes (De Lemos et al., 2003; Liuzzo et al., 1994; Liuzzo et al., 1999).

Measurement of serum troponin as introduced into clinical practice almost 20 years ago, has lead to a major improvement in the detection of patients with ruptured plaques (Cummins et al., 1987; Katus et al., 1989). Troponin is a highly sensitive and specific marker of myocardial necrosis. Accordingly, the determination of troponin levels is established as being a valuable tool for the analysis of myocardial infarction and has become a routine step for the physician.

However, an essential and yet unresolved problem is the diagnostic gap to discriminate non cardiac chest pain or symptoms due to stable CAD from an ACS or an impending MI, which does not allow a separation of patients presenting in the emergency room in those having stable CAD and those with unstable CAD with plaque erosion, plaque rupture, coronary thrombosis and the risk of consequent serious cardiovascular events such as MI.

Troponin itself is not originating from atherosclerotic lesions. Its detection in serum may reflect microscopic infarcts due to embolization of lesion content and thrombotic material into the cardiac microcirculation and therefore, troponin may also be indicative of plaque rupture. But troponin is only an indirect indicator of plaque rupture reflecting already ongoing myocardial necrosis rather than ruptured coronary lesions. Indeed, a number of patients initially presenting with chest pain and repeatedly negative troponin have major cardiac events such as myocardial infarction or death within the next 30 days (Hamm et al., 1997).

Indicators of myocardial necrosis as markers of ACS have also the disadvantage that they need at least 2-3 hours from the onset of symptoms in order to be detectable and furthermore represent myocardial damage, which has negative impact on prognoses (Newby et al., 2001; Alpert et al., 2000).

There is an urgent clinical demand for an early and more specific marker of local inflammatory processes which allows an early diagnosis of an ACS or an impending MI, and separation of patients presenting in the emergency room with either non cardiac chest pain or symptoms due to stable CAD. Indeed, immediate coronary angiography and primary percutaneous coronary intervention has been established as the undisputed therapy of choice in ACS and MI (Widimsky et al., 2003), whereas its extension to patients with stable CAD and atypical chest pain represents inadequate allocation of expensive emergency resources.

WO 2004/057341 discloses the protein calprotectin as a useful marker or indicator of potential for or propensity to a cardiovascular disease before the onset of cardiovascular disease symptoms, i.e. in symptom-free subjects. Calprotectin also termed as myeloid-related protein 8/14 complex (MRP 8/14), leukocyte protein L1 complex, migration inhibitory factor (MIF)-related protein or cystic fibrosis antigen is a hetero-dimer of two calcium-binding proteins S100A8 and S100A9 (also termed as MRP8 and MRP14 or calcranulin A and B). It is a marker of phagocyte activation (Murao et al., 1994; Hansson, 2005) with a variety of functions including calcium-dependent signaling, cell differentiation, cell cycle progression, and cytoskeleton-membrane interactions (Schafer et al., 1996). MRP8 and MRP 14 are mainly expressed in cells of myeloid origin, comprising 1% of all cytosolic proteins in monocytes and up to 30-60% in neutrophils (Hessian et al., 1993). However, upon activation of these phagocytes, which leads to an increase in intracellular calcium and activation of protein kinase C, pre-existing cytoplasmatic MRP8 and MRP 14 can rapidly form MRP 8/14, which translocates to the cytoskeleton as well as plasma membranes, and finally gets secreted (Teigelkamp et al., 1991; Rammes et al., 1997; Stroncek et al., 2005). This typically occurs during transendothelial migration and interaction of MRP 8/14 expressing phagocytes such as circulating neutrophils and early differentiation stages of monocytes, but not of resting tissue macrophages, with activated endothelium (Odink et al., 1987; Bhardwaj et al., 1992; Eue et al., 2000; Frosch et al., 2000). As a consequence MRP 8/14 has proven to be an indicator of minimal inflammatory activity, providing additional information not accessible by other markers of inflammation (Foell et al., 2004). Especially in non-infectious inflammatory diseases such as arthritis, chronic inflammatory lung and bowel disease as well as acute renal allograft rejection, MRP 8/14 is a very sensitive parameter for the monitoring of disease activity and response to treatment (De Rycke et al., 2005; Roth et al., 1992; Rugveit et al., 1994; Burkhardt et al., 2001). According to WO 2004/057341, it has been found that abnormally high MRP 8/14 levels in various body fluids are indicative of susceptibility to cardiovascular disease before the onset of cardiovascular disease symptoms is apparent to a subject or to a third party, such as a physician.

Healy et al. (2006) describe the expression profiling of platelet mRNA from patients with acute ST-segment-elevation myocardial infarction (STEMI) or stable coronary artery disease. The authors found that MRP 14 expression - which represents another biochemical entity than the heterodimer MRP 8/14 - increases before STEMI and, taking a prospective, nested case-control study (n=255 case-control pairs) among apparently healthy women into account, they found that increasing plasma concentrations of - now the heterodimer - MRP 8/14 among healthy individuals predict the risk of future cardiovascular events. However, neither WO 2004/057341 nor Healy et al. (2006), which can be considered as the closest prior art for the present invention, discloses MRP 8/14 as a marker to determine whether an individual presenting with chest pain is already suffering from an ACS and, as a consequence, will develop a cardiovascular event in the near future. Rather, both contestants claim a general risk stratification and do not show data documenting the discrimination of patients presenting with symptoms of an acute coronary syndrome in progress to myocardial infarction from patients with stable coronary artery disease or absence of coronary pathology.

Hence, the technical problem underlying the present invention is to provide a method using an early and sensitive marker of local vascular processes with the potential to identify ACS, i.e. for example plaque instability in individuals showing early symptoms of a CHD, either before irreversible clinical events have occurred and serum markers of myocardial necrosis are detectable, or on condition that such events already have occurred, to detect them with higher sensitivity and earlier than with any known marker. A method using such an early and sensitive marker should allow to rule out the likelihood and the risk, respectively, whether an individual showing one or more symptoms of an evolving coronary syndrome is suffering from an acute coronary syndrome. Accordingly, the individual would benefit from an adequate treatment as soon as necessary. This would contribute to economic resource utilization in health care.

### Summary of the invention

The object of the present invention is a method for determining the risk whether an individual showing at least one symptom of an evolving acute coronary syndrome is suffering from an acute coronary syndrome comprising the steps of (a) measuring, preferably in vitro, the level of MRP 8/14, wherein (b) if the level of MRP 8/14 is at least increased, then the individual is at risk of suffering from an acute coronary syndrome. Also within the scope of the present invention is a method to rule out the likelihood whether an individual showing at least one symptom of an evolving acute coronary syndrome is suffering from an acute coronary syndrome. Another object of the present invention is a method to assess whether an individual showing at least one symptom of an evolving acute coronary syndrome is not at risk of suffering from an acute coronary syndrome and a method to discriminate if an individual showing at least one symptom of an evolving acute coronary syndrome is at risk to suffer from an acute coronary syndrome and/or has no acute coronary syndrome.

Also within the scope of the present invention is a kit comprising a means or an agent for measuring MRP 8/14, wherein the kit comprises a user's manual for interpreting the results of any measurement(s) with respect to determining the risk of an individual of suffering from an acute coronary syndrome, and the use of a kit comprising a means or an agent for measuring MRP 8/14, for determining the risk of an individual of suffering from an acute coronary syndrome. The present invention assists physicians in rapidly discriminating which individuals suffering from chest pain and/or any pertinent clinical symptoms have an acute coronary syndrome before markers of myocardial necrosis are detectable. Due to the new use of this diagnostic marker these individuals will preferentially benefit from rapid and early treatment, leading to improved outcome.

### Brief description of the drawings

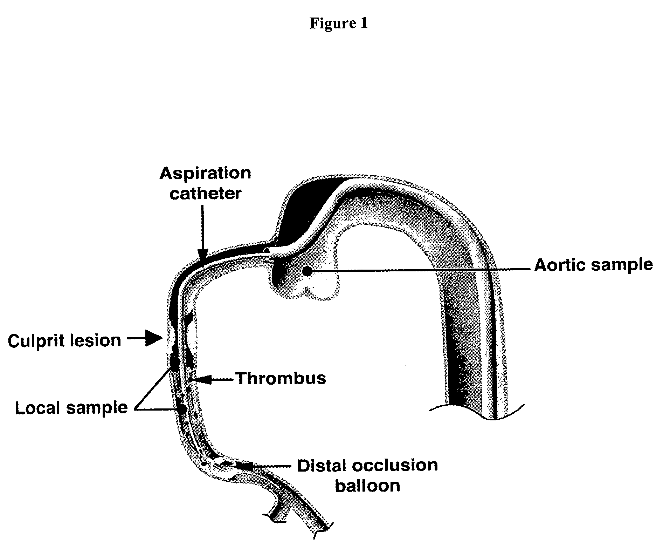

- Figure 1:: Systemic and local sampling with a temporary occlusion and aspiration system in patients with ACS.

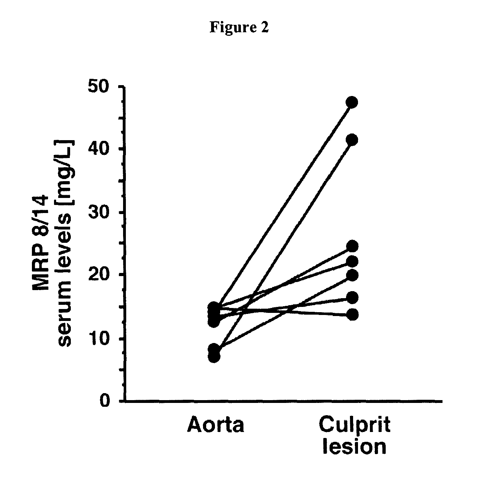

- Figure 2:: Serum concentrations of MRP 8/14 at the site of the culprit lesion compared with systemic values taken from the aorta. Local levels are significantly elevated (n=7; p<0.05). Culprit lesions are the predominant lesions responsible for an ACS. Culprit lesions result as a consequence of plaque rupture, plaque erosion and/or thrombus formation.

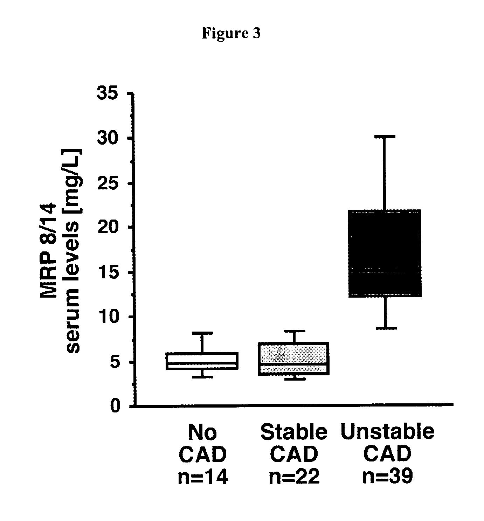

- Figure 3:: Systemic concentrations (aorta) of MRP 8/14 in three different groups of patients classified according to coronary angiography: Left, patients without evidence of coronary artery disease (no CAD). Middle, patients with stable coronary artery disease (stable CAD). Right, patients with coronary artery disease and at least one clearly identifiable culprit lesion, as defined by morphological aspects, signs of intra-coronary thrombosis, and local flow impairment (unstable CAD). Patients with unstable coronary artery disease show significantly elevated levels of MRP 8/14 compared with the other two groups.

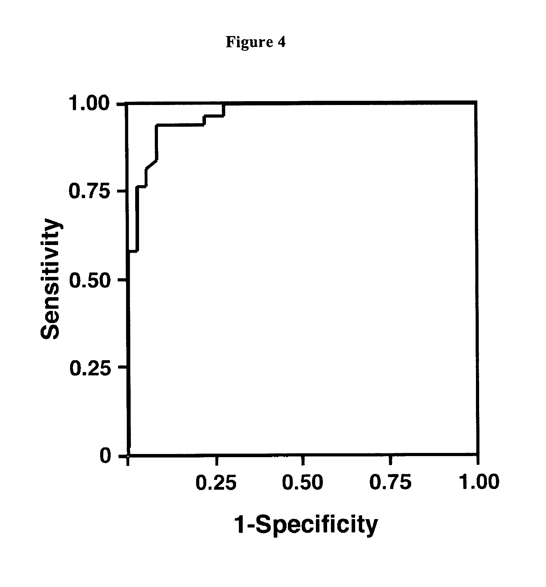

- Figure 4:: Receiver-operating-characteristic (ROC) for MRP 8/14 to detect patients being at risk of an acute coronary syndrome. The ROC curve is a common method to quantify the accuracy of a medical test. It indicates the probability of a true positive result as a function of the probability of a false positive result for all possible threshold values. The ROC curve provides two important indicators of the accuracy of a test: the cut-off value (threshold value with both highest sensitivity and specificity) and the area under the curve (AUC). For MRP 8/14 the cut-off level is 8.0 mg/L with a sensitivity of 95% and a specificity of 92%. The AUC is 0.97.

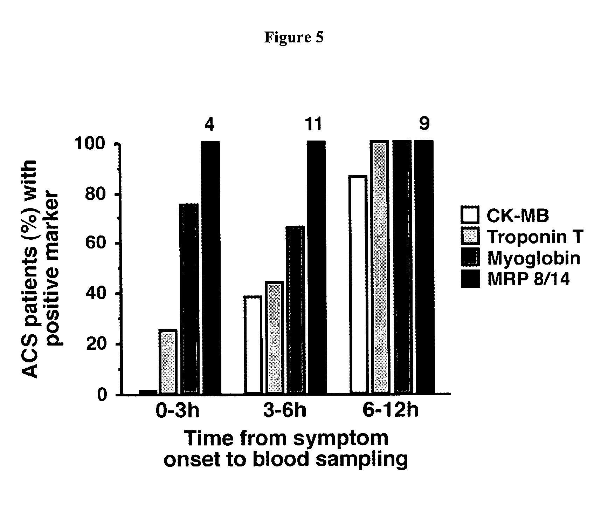

- Figure 5:: Percentage of ACS patients presenting with elevated levels of MRP 8/14 and classical markers of myocardial necrosis in relation to the time (h; hours) from symptom onset to blood sampling. The numbers of patients for the three different time intervals are 4, 11, and 9, respectively.

### Detailed description of the invention

Surprisingly, it has been found that the myeloid-related protein 8/14 complex (MRP 8/14) can be used to identify those patients among individuals showing any symptoms of an evolving acute coronary syndrome (ACS), who have a likelihood to suffer from an ACS, thereby ruling out those individuals having no ACS. This e.g. allows a separation of patients presenting in the emergency room in those having a stable coronary artery disease (CAD) or non cardiac chest pain with normal coronary arteries and those who are suffering from ACS and potentially further serious cardiovascular events such as MI.

The object of the invention is attained by a method for determining the risk whether an individual showing at least one symptom of an evolving ACS is suffering from an ACS, comprising the steps of
a. measuring, preferably in vitro, the level of MRP 8/14 or a variant thereof, wherein
b. if the level of MRP 8/14 or a variant thereof is at least increased, then the individual is at risk of suffering from an ACS.

Furthermore, the object of the invention is attained by a method for determining the risk whether an individual showing at least one symptom of an evolving ACS is suffering from an ACS, comprising the steps of
a. measuring, preferably in vitro, the level of MRP 8/14 or a variant thereof,
aa. measuring, preferably in vitro, the level of markers of myocardial necrosis and/or inflammatory markers and/or metalloproteinases or a variant thereof, wherein
ba. if the levels of both the markers of myocardial necrosis and/or inflammatory markers and/or metalloproteinases and MRP 8/14, or a respective variant thereof, are at least increased, then the individual is at least at risk of suffering from an ACS.

An embodiment of the present invention comprises a method for determining a risk whether an individual showing at least one symptom of an evolving acute coronary syndrome is suffering from an acute coronary syndrome, comprising the steps of
a. measuring, preferably in vitro, the level of MRP 8/14 or a variant thereof, wherein
b. if the level of MRP 8/14 or a variant thereof corresponds to a serum level of at least 8.0 mg/L, then the individual is at risk of suffering from an acute coronary syndrome.

The term "symptom of an evolving ACS" is known to person skilled in the art. An individual "showing at least one symptom of an evolving ACS" means e.g. an individual presenting with chest pain at rest, ongoing or undulating, (constricting, crushing, oppressing, compressing, choking, vise-like), sometimes in combination with symptoms of an indisgestion (Messerli et al., 2005), nausea, vomiting, weakness, dizziness, palpitations, cold perspiration, breathlessness, but also individuals rather presenting with chest pain manifestations which are in general considered as atypical (sharp or knife-like pain, pain that may be localized at the tip of one finger, pain reproduced with movement of the chest, constant pain that persists for many hours, very brief episodes of pain that last a few seconds or less, pain that radiates into the lower extremities). The latter pain conditions need to be evaluated as carefully as typical pain. The patient may present in private practice or at the emergency room. The terms "showing a symptom", "suffering from a symptom" as well as other synonyms for having a symptom are contemplated within the scope of the present invention.

An embodiment of the present invention relates to a method for determining the risk whether an individual showing breathlessness and/or chest pain is suffering from an ACS, comprising the steps of,
a. measuring, preferably in vitro, the level of MRP 8/14 or a variant thereof, wherein
b. if the level of MRP 8/14 or a variant thereof is at least increased, then the individual is at risk of suffering from an ACS.

According to the present invention, the level of MRP 8/14 measured in an individual allows to determine a risk of whether an individual is likely to suffer from an ACS. More particularly, an increased level of MRP 8/14 indicates that the respective individual is at risk of suffering from an ACS. On the other hand, a level of MRP 8/14, which is not elevated, indicates that the respective individual is not at risk of suffering from an ACS, i.e. the respective individual does not suffer from an ACS or has no ACS, respectively.

Thus, by measuring the level of MRP 8/14 an individual can be assigned to a given risk group, e.g. those individuals having a risk of suffering from ACS and those individuals who do not suffer from ACS.

Accordingly, another aspect of the present invention is a method to rule out the likelihood whether an individual showing at least one symptom of an evolving ACS is suffering from an ACS, comprising the steps of
a. measuring, preferably in vitro, the level of MRP 8/14 or a variant thereof, wherein
b. if the level of MRP 8/14 or a variant thereof is not elevated, then the individual does not suffer from an ACS.

Furthermore, the object of the invention is attained by a method to assess whether an individual showing at least one symptom of an evolving ACS is not at risk of suffering from an ACS, comprising the steps of
a. measuring, preferably in vitro, the level of MRP 8/14 or a variant thereof, wherein
b. if the level of MRP 8/14 or a variant thereof is not elevated, then the individual is not at risk of suffering from an ACS.

Also within the scope of the present invention is a method to discriminate if an individual showing at least one symptom of an evolving ACS is at risk to suffer from an ACS and or has no ACS, comprising the steps of
a. measuring, preferably in vitro, the level of MRP 8/14 or a variant thereof, wherein
b. if the level of MRP 8/14 or a variant thereof is at least increased, then the individual is at risk of suffering from an ACS,
c. if the level of MRP 8/14 or a variant thereof is not elevated, then the individual has no ACS.

An embodiment of the present invention relates to any of the methods as mentioned before, wherein a not elevated level of MRP 8/14 corresponds to a serum level of less than 8.0 mg/L.

A further aspect of the present invention is the use of MRP 8/14 or a variant thereof for determining the risk whether an individual showing at least one symptom of an evolving ACS is suffering from an ACS, comprising the steps of
a. measuring, preferably in vitro, the level of MRP 8/14 or a variant thereof, wherein
b. if the level of MRP 8/14 or a variant thereof is at least increased, then the individual is at risk of suffering from an ACS.

Another aspect of the present invention is the use of MRP 8/14 or a variant thereof to rule out the likelihood whether an individual showing at least one symptom of an evolving ACS is suffering from an ACS, comprising the steps of
a. measuring, preferably in vitro, the level of MRP 8/14 or a variant thereof, wherein
b. if the level of MRP 8/14 or a variant thereof is not elevated, then the individual does not suffer from an acute coronary syndrome.

Also within the scope of the present invention is one of the methods or the use of MRP 8/14 as mentioned before, wherein if the method indicates that the risk is at least increased, it indicates that intensive coronary care should be performed without undue delay and/or in a hospital setting.

Intensive coronary care includes e.g. cardiac monitoring, serial assessments of ECG and biochemical parameters, standard medication (such as aspirin, nitrates, analgetics, clopidogrel etc.), coronary angiography, and percutaneous coronary intervention, if suitable.

Also within the scope of the present invention is one of the methods or the use of MRP 8/14 as mentioned before, wherein if the method indicates that the individual has no ACS, it indicates that the individual may be treated otherwise, e.g. may be assigned to elective non invasive differential diagnostics of chest pain (e.g. exercise stress testing) possibly at his local general practitioner. I.e. the individual will not undergo intensive coronary care, e.g. the individual may be subjected to further elective, non cardiac or cardiac examinations as soon as possible. Thus the individual will benefit from an adequate treatment as soon as necessary. This will contribute to economic resource utilization in health care.

In the context of the present invention, it has been realized that the level of MRP 8/14 provides information indicating a risk of suffering from an ACS. In particular, it has been realized that, if the level of MRP 8/14 is not elevated, the risk that an individual is suffering from an ACS can be ruled out.

It has been realized that, in contrast to the levels of indicators of myocardial necrosis such as troponin, the level of MRP 8/14 is dependent on local processes preceding and accompanying plaque rupture and early thrombus formation. In atherosclerosis and especially in the pathogenic chain of stable atherosclerotic plaques progressing into vulnerable lesions, recruitment of inflammatory cells, notably phagocytes expressing MRP 8/14 is crucial. Accordingly, the level of MRP 8/14 appears to indicate whether active vascular inflammatory processes irrespective of the global atherosclerotic burden are present in an individual. The present invention has realized that it is possible to take advantage of the information provided by the level of MRP 8/14 in the context of risk prediction.

In contrast, the levels of indicators of myocardial necrosis such as troponin are only indirect indicators of plaque rupture reflecting already ongoing myocardial necrosis rather than ruptured coronary lesions. It should also be noted that there is a number of patients initially presenting with chest pain and repeatedly negative troponin and having major cardiac events such as myocardial infarction or death within the next 30 days (Hamm et al., 1997). Further, indicators of myocardial necrosis as markers of ACS have also the disadvantage that they need at least 2-3 hours from the onset of symptoms in order to be detectable and furthermore represent myocardial damage, which has negative impact on prognoses (Newby et al., 2001; Alpert et al., 2000). Thus, measuring the levels of indicators of myocardial necrosis such as troponin alone may result in a number of false negative results regarding the risk for an ACS. However, in the context of the present invention, it has been realized that the levels of MRP 8/14 and markers of myocardial necrosis and/or inflammatory markers and/or metalloproteinases can be measured in combination, simultaneously or nonsimultaneously, in order to determine a risk whether an individual will suffer from an ACS. It has been realized that the information provided by MRP 8/14 and markers of myocardial necrosis and/or inflammatory markers and/or metalloproteinases may complement each other advantageously and thus may provide an improved risk determination.

Advantageously, the present invention will allow to assign individuals to different settings of further treatment, namely intensive coronary care. E.g. individuals for whom the present invention indicates an increased risk will preferably be further treated in a setting providing improved precautions for intervention in case of an ACS. Such individuals also may be treated e.g. by cardiac monitoring, serial assessments of ECG and biochemical parameters, standard medication (such as aspirin, nitrates, analgetics, clopidogrel etc.), coronary angiography and percutaneous coronary intervention, if suitable to reduce the risk of suffering from an ACS.

In contrast, an individual for whom a method according to the present invention indicates no risk of suffering from ACS may be treated otherwise, e.g. may be assigned to elective non invasive differential diagnostics of chest pain (e.g. exercise stress testing) possibly at his local general practitioner. Thus the individual will benefit from an adequate treatment as soon as necessary. This will contribute to economic resource utilization in health care, thus last but not least, leading to significant cost-savings in the health care system.

The invention takes advantage of certain biomarkers, in particular "biochemical markers" and "molecular markers". Biomarkers are known to the person skilled in the art. The term relates to molecules in an individual which are differentially present (i.e. present in increased or decreased levels) depending on presence or absence of a certain condition, disease, or complication. The terms "biochemical marker" and "molecular marker" are known to the person skilled in the art. In particular, biochemical or molecular markers are gene expression products which are differentially present (e.g. through increased or decreased level of expression or turnover) in presence or absence of a certain condition, disease, or complication. Usually, a molecular marker is defined as a nucleic acid (such as an mRNA), whereas a biochemical marker is a protein or peptide. The level of a suitable biomarker can indicate the presence or absence of a particular condition, disease, or risk, and thus allow diagnosis or determination of the condition, disease, or risk.

The present invention particularly takes advantage of MRP8/14 and markers of myocardial necrosis and/or inflammatory markers and/or metalloproteinases as biochemical markers.

The myeloid-related protein 8/14 complex (MRP 8/14) is well-known to the person skilled in the art. MRP 8/14 also known as calprotectin, leukocyte protein L1 complex, migration inhibitory factor (MIF)-related protein or cystic fibrosis antigen exists in both dimeric and multimeric forms. As a dimer, MRP 8/14 comprises the polypeptide chains S100A8 and S100A9 (also termed as MRP8 and MRP 14 or calcranulin A and B). As a trimer, MRP 8/14 is a 36 kDa heterotrimeric protein with two heavy (14 kD) and one light chain (8kD) being non-covalently linked. MRP 8/14 is a marker of phagocyte activation (Murao et al., 1994; Hansson, 2005) with a variety of functions including calcium-dependent signaling, cell differentiation, cell cycle progression, and cytoskeleton-membrane interactions (Schafer et al., 1996). MRP8 and MRP14 are mainly expressed in cells of myeloid origin, comprising 1% of all cytosolic proteins in monocytes and up to 30-60% in neutrophils (Hessian et al., 1993).

Markers of myocardial necrosis and/or inflammatory markers and/or metalloproteinases according to the present invention comprise myoglobin, creatine kinase, creatine kinase-MB (CK-MB), troponin T and I, C-reactive protein (CRP), interleukin 6 (I1-6), serum amyloid A (SAA), myeloperoxidase, soluble CD40 ligand (sCD40L), and pregnancy-associated plasma protein A (PAPP-A). The most preferred markers according to the present invention are myoglobin, creatinkinase, CK-MB, troponin T and troponin I.

The term "variants" in the context of the present invention relates to proteins or peptides substantially similar to said proteins or peptides. The term "substantially similar" is well understood by the person skilled in the art. In particular, a variant may be an isoform or allele which shows amino acid exchanges compared to the amino acid sequence of the most prevalent peptide isoform in the human population. Preferably, such a substantially similar peptide has a sequence similarity to the most prevalent isoform of the protein or peptide of at least 80%, preferably at least 85%, more preferably at least 90%, most preferably at least 95%. Substantially similar are also degradation products, e.g. proteolytic degradation products, which are still recognized by the diagnostic means or by ligands directed against the respective full-length protein or peptide. The term "variants" is also meant to relate to splice variants. The term "variant" also relates to a post-translationally modified peptide such as glycosylated peptide. A "variant" is also a peptide which has been modified after collection of the sample, for example by covalent or non-covalent attachment of a label, particularly a radioactive or fluorescent label, to the peptide.

The invention also includes the measuring of different markers in combination, simultaneously or non-simultaneously. In particular, the present invention relates to measuring MRP 8/14 in combination with markers of myocardial necrosis and/or inflammatory markers and/or metalloproteinases, in particular myoglobin, creatinkinase, CK-MB, troponin T and troponin I. According to the present invention any further markers may be measured in combination with MRP 8/14. Examples for such markers include myoglobin, creatinkinase, CK-MB, troponin T and I, CRP, II-6, SAA, myeloperoxidase, sCD40L, and PAPP-A.

The present invention allows to determine a risk (or to predict) whether an indivicual showing at least one symptom of an evolving ACS is suffering from an ACS. The term ACS ("acute coronary syndrome") is known to the person skilled in the art. In the context of the present invention, the term ACS includes unstable angina pectoris, non-STEMI, and STEMI.

An embodiment of the present invention relates to one of the methods or the use of MRP 8/14 as mentioned before, wherein the ACS is an ACS occurring as a consequence of a vascular event such as an atherosclerotic plaque rupture, an atherosclerotic plaque erosion and/or a thrombus formation.

The term "atherosclerotic plaque rupture" (which, in the context of the present invention, is also simply referred to as "plaque rupture"), as well as the terms "atherosclerotic plaque erosion" and "thrombus formation" are known to the person skilled in the art. An atherosclerotic plaque contains as a substantial part an abnormal inflammatory accumulation of macrophages and other white blood cells within the walls of arteries. These anatomic lesions typically begin in later childhood, well before age 10, and progress over time. Veins do not develop atheromata, unless surgically moved to function as an artery, as in bypass surgery. The accumulation is always between the endothelium lining and the smooth muscle wall of the arterial tube. While the early stages, based on gross appearance, have traditionally been termed fatty streaks by pathologists, they are not composed of fat, i.e. adipose cells. In the context of heart or artery matters, atherosclerotic plaques are commonly referred to as "plaques". Pathologically, the atherosclerotic plaque is divided into three distinct components: 1. The atheroma ("lump of porridge", from Athera, porridge in Greek,) is the nodular accumulation of a soft, flaky, yellowish material at the center of large plaques, composed of macrophages nearest the lumen of the artery, sometimes with 2. underlying areas of cholesterol crystals and possibly also 3. calcification at the outer base of older/more advanced lesions. Angiographic studies have identified culprit lesions that do not cause marked stenosis, and it is now evident, that the activation of plaque with the formation of a superimposed thrombus rather than stenosis precipitates infarction. In the majority of cases, formation of an occluding thrombus arises from plaque rupture, exposing thrombotic material from the core of the plaque (phospholipids, tissue factor, and platelet-adhesive matrix molecules) to blood. Another cause of coronary thrombi are endothelial erosions, plaque fissuring, and in some cases coronary spasms may be involved. Plaque ruptures preferentially take place where the fibrous cap of the atheroma is thin and activated cells of the immune system are abundant. In such sites, the combined release of inflammatory mediators and proteolytic enzymes from activated immune cells may weaken the fibrous cap and transform a stable plaque into a vulnerable, unstable plaque, that can rupture, induce a coronary thrombus, and elicit an ACS. The present invention also allows to rule out the likelihood whether an individual showing at least one symptom of an evolving ACS is suffering from ACS or to discriminate if an individual showing at least one symptom of an evolving ACS is at risk to suffer from an ACS or has no ACS.

An individual having no ACS may be an individual showing at least one symptom of an evolving ACS and having e.g. pericardial, musculoskeletal, gastrointestinal, pulmonary, psycho-vegetative, and psychiatric syndromes, in particular without underlying acute inflammatory disease. Such an individual will undergo related diagnostics (e.g. gastroscopy or CT-scan) and an adequate treatment, which is known to a skilled person, such as reassurance and pharmacological sedation in case of psycho-vegetative symptoms. If the level of MRP 8/14 is not elevated, patient will not undergo intensive coronary care, but may be subjected to further elective, non cardiac or cardiac examinations according to their symptoms.

An individual having no ACS also may be an individual showing at least one symptom of an evolving ACS and not suffering from any condition, i.e. a healthy individual showing at least one symptom of an evolving ACS.

The above definition of the term "an individual having no ACS" also applies to an individual not suffering from ACS and an individual not being at risk for ACS.

It has also been found, that the present invention provides diagnostic information as such. For example, if a method according to the present invention indicates an increased risk for ACS, then the individual may be subjected to further intensive coronary care. On the other hand, if a method according to the present invention indicates no risk for ACS, then the individual may be treated as indicated above.

The individual according to the present invention can be any individual or patient who has experienced (or is currently experiencing) symptoms of a cardiovascular dysfunction, e.g. chest pain (angina pectoris), shortness of breath (dyspnea), palpitation. Particularly, patient may be an individual showing symptoms, which would classify him as having angina pectoris according to the Canadian Cardiovascular Society (CCS) classification of stable angina pectoris (Campeau, 1976) and/or the Braunwald and Hamm classification of unstable angina pectoris (Braunwald, 1989; Hamm and Braunwald, 2000).

The CCS classification discriminates 4 classes, in detail as follows. Class I: Ordinary physical activity does not cause angina, such as walking, climbing stairs. Angina occurs with strenuous, rapid, or prolonged exertion at work or recreation. Class II: Slight limitation of ordinary activity. Angina occurs on walking or climbing stairs rapidly, walking uphill, walking or stair climbing after meals, or in cold, or in wind, or under emotional stress, or only during the few hours after awakening. Walking more than two blocks on the level and climbing more than one flight of ordinary stairs at a normal pace and in normal condition. Class III: Marked limitations of ordinary physical activity. Angina occurs on walking one to two blocks on the level and climbing one flight of stairs in normal conditions and at a normal pace. Class IV: Inability to carry on any physical activity without discomfort -- angina symptoms may be present at rest. Patients with ACS usually present as CCS Class IV.

The Braunwald and Hamm classification of unstable angina pectoris distinguishes 3 classes and 3 associated clinical circumstances, in detail as follows. Class I: New onset severe or accelerated angina. Patients with new onset (<2 months in duration) exertional angina pectoris that is severe or frequent (>3 episodes/day) or patients with chronic stable angina who develop accelerated angina (that is, angina distinctly more frequent, severe, longer in duration, or precipitated by distinctly less exertion than previously) but who have no experienced pain at rest during the preceding 2 months. Class II: Angina at rest, subacute. Patients with one or more episodes of angina at rest during the preceding month but not within the preceding 48 hours. Class III: Angina at rest, acute. Patients with one or more episodes of angina at rest within the preceding 48 hours. In classes II and III, manifestations described in class I may also occur. Unstable angina is no longer considered to be present when a patient has been asymptomatic or suffers angina that has been stable for more than 2 months. The associated clinical circumstances, in which unstable angina occurs, are divided into classes A-C, in detail as follows. Class A: Secondary unstable angina. Patients in whom unstable angina develops secondary to a clearly identified condition extrinsic to the coronary vascular bed that has intensified myocardial ischemia. Such conditions reduce myocardial oxygen supply or increase myocardial oxygen demand and include anemia, fever, infection, hypotension, uncontrolled hypertension, tachyarrhythmia, unusual emotional stress, thyrotoxicosis, and hypoxemia secondary to respiratory failure. Class B: Primary unstable angina. Patients who develop unstable angina pectoris in the absence of an extracardiac condition that has intensified ischemia, as in class A. Class C: Postinfarction unstable angina. Patients who develop unstable angina within the first 2 weeks after a documented acute myocardial infarction.

There is a particular need to rule in patients presenting with symptoms according to the Braunwald and Hamm classification and having an ACS for further diagnostics and therapy, and to rule out patients presenting with symptoms mistakable for the Braunwald and Hamm classification and not having an ACS.

Advantageously, the invention allows to detect patients having a risk in these patient populations. In samples obtained from such patients MRP 8/14 should preferably be measured in order to determine the risk for an ACS.

Methods and diagnostic means which can be used to determine the levels of the respective peptides are known to the person skilled in the art. These methods include microplate ELISA-based methods, fully-automated or robotic immunoassays (available for example on ElecsysTM analyzers), CBA (an enzymatic Cobalt Binding Assay, available for example on Roche-HitachiTM analyzers), and latex agglutination assays (available for example on Roche-HitachiTM analyzers).

Furthermore, the person skilled in the art is familiar with different methods of measuring the level of a peptide or polypeptide. The term "level" relates to amount or concentration of a peptide or polypeptide in an individual or a sample taken from an individual.

The term "measuring" according to the present invention relates to determining the amount or concentration, preferably semi-quantitatively or quantitatively, protein, peptide, polypeptide, or other substance of interest. Proteins or peptides of particular interest in the context of the present invention are the myeloid-related protein 8/14 complex (MRP 8/14) and markers of myocardial necrosis and/or inflammatory markers and/or metalloproteinases. The level of MRP 8/14 and markers of myocardial necrosis and/or inflammatory markers and/or metalloproteinases can be measured by quantitative, semi-quantitative and qualitative methods, as well as by all other methods for measuring MRP 8/14 and markers of myocardial necrosis and/or inflammatory markers and/or metalloproteinases. For example, a method that merely detects the presence or absence of MRP 8/14 and markers of myocardial necrosis and/or inflammatory markers and/or metalloproteinases in a sample suspected of containing MRP 8/14 and markers of myocardial necrosis and/or inflammatory markers and/or metalloproteinases is considered to be included within the scope of the present invention. The terms "detecting", "monitoring" and "determining", as well as other common synonyms for measuring, are contemplated within the scope of the present invention.

Measuring can be done directly or indirectly. Indirect measuring includes measuring of cellular responses, bound ligands, labels, or enzymatic reaction products.

In the context of the present invention, amount also relates to concentration. It is evident, that from the total amount of a substance of interest in a sample of known size, the concentration of the substance can be calculated, and vice versa.

Measuring can be done according to any method known in the art. Preferred methods are described in the following.

In a preferred embodiment, the method for measuring the level of a protein, peptide, or polypeptide of interest, comprises the steps of (a) contacting a cell capable of a cellular response to the protein, peptide or polypeptide with the protein, peptide or polypeptide for an adequate period of time, (b) measuring the cellular response.

In another preferred embodiment, the method for measuring the level of a protein, peptide or polypeptide of interest, comprises the steps of (a) contacting a protein, peptide or polypeptide with a suitable substrate for an adequate period of time, (b) measuring the amount of product.

In another preferred embodiment, the method for measuring the level of a peptide or polypeptide of interest, comprises the steps of (a) contacting a protein, peptide or polypeptide with a specifically binding ligand, (b) (optionally) removing non-bound ligand, (c) measuring the amount of bound ligand.

Preferably, the protein, peptide or polypeptide is contained in a sample, particularly a body fluid or tissue sample, and the amount of the protein, peptide or polypeptide in the sample is measured.

Proteins, peptides and polypeptides can be measured in tissue, cell, and body fluid samples, i.e. preferably in vitro. Preferably, the protein, peptide or polypeptide of interest is measured in a body fluid sample.

A tissue sample according to the present invention refers to any kind of tissue obtained from the dead or alive human or animal body. Tissue samples can be obtained by any method known to the person skilled in the art, for example by biopsy or curettage.

Body fluids according to the present invention may include blood, blood serum, blood plasma, lymphe, cerebral liquor, saliva, and urine. Particularly, body fluids include blood, blood serum, blood plasma, and urine. An embodiment of the present invention relates to the measurement ofMRP 8/14 in blood, preferably in serum and/or plasma. The sample can be pretreated if desired and can be prepared in any convenient medium that does not interfere with the measurement. Samples of body fluids can be obtained by any method known in the art.

Another embodiment of the present invention relates to a method for determining the risk whether an individual will suffer from an ACS, comprising the steps of
a. measuring the level of MRP 8/14 or a variant thereof in serum and/or plasma, preferably extracted from blood of peripheral circulation wherein
b. if the level of MRP 8/14 or a variant thereof is at least increased, then the individual is at least at risk of suffering from an ACS.

Methods to obtain cell samples include directly preparing single cells or small cell groups, dissociating tissue (e.g. using trypsin or collagenases), and separating cells from body fluids, e.g. by filtration or centrifugation. Cells according to the present invention comprise also platelets and other non-nuclear cells, e.g. erythrocytes. If necessary, the samples may be further processed. Particularly, proteins, peptides or polypeptides may be purified from the sample according to methods known in the art, including filtration, centrifugation, or extraction methods such as chloroform/phenol extraction.

For measuring cellular responses, the sample or processed sample is added to a cell culture and an internal or external cellular response is measured. The cellular response may include the expression of a reporter gene or the secretion of a substance, e.g. a protein, peptide, polypeptide, or a small molecule.

Other preferred methods for measurement may include measuring the amount of a ligand binding specifically to the protein, peptide or polypeptide of interest. Binding according to the present invention includes both covalent and non-covalent binding. A ligand according to the present invention can be any protein, peptide, polypeptide, nucleic acid, or other substance binding to the protein, peptide or polypeptide of interest. It is well known that proteins, peptides or polypeptides, if obtained or purified from the human or animal body, can be modified, e.g. by glycosylation. A suitable ligand according to the present invention may bind the protein, peptide or polypeptide exclusively or additionally via such sites.

Preferably, the ligand should bind specifically to the protein, peptide or polypeptide to be measured. "Specific binding" according to the present invention means that the ligand should not bind substantially to ("cross-react" with) another protein, peptide, polypeptide or substance present in the sample investigated. Preferably, the specifically bound protein or isoform should be bound with at least 3 times higher, more preferably at least 10 times higher and even more preferably at least 50 times higher affinity than any other relevant protein, peptide or polypeptide.

Non-specific binding may be tolerable, particularly if the investigated protein, peptide or polypeptide can still be distinguished and measured unequivocally, e.g. according to its size (such as on a Western Blot), or by its relatively higher abundance in the sample.

Binding of the ligand can be measured by any method known in the art. Preferably, the method is semi-quantitative or quantitative. Suitable methods are described in the following.

First, binding of a ligand may be measured directly, e.g. by NMR or surface plasmon resonance.

Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot).

For measurement of enzymatic reaction products, preferably the amount of substrate is saturating. The substrate may also be labeled with a detectable label prior to the reaction. Preferably, the sample is contacted with the substrate for an adequate period of time. An adequate period of time refers to the time necessary for an detectable, preferably measurable amount of product to be produced. Instead of measuring the amount of product, the time necessary for appearance of a given (e.g. detectable) amount of product can be measured.

Third, the ligand may be coupled covalently or non-covalently to a label allowing detection and measurement of the ligand.

Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.)

The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus.

Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as readymade stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously.

Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molcular Probes (Oregon). Also the use of quantum dots as fluorescent labels is contemplated.

Typical radioactive labels include 35S, 125I, 32P, 33P and the like. A radioactive label can be detected by any method known and appropriate, e.g. a light-sensitive film or a phosphor imager.

Suitable measurement methods according the present invention also include precipitation (particularly immunoprecipitation), electrochemiluminescence (electrogenerated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latexenhanced turbidimetry or nephelometry, solid phase immune tests, and mass spectrometry such as SELDI-TOF, MALDI-TOF, or capillary electrophoresis-mass spectrometry (CEMS). Further methods known in the art (such as gel electrophoresis, 2D gel electrophoresis, SDS polyacrylamid gel electrophoresis (SDS-PAGE), Western Blotting), can be used alone or in combination with labeling or other detection methods as described above.

Preferred ligands include antibodies, nucleic acids, proteins, peptides or polypeptides, and aptamers, e.g. nucleic acid or peptide aptamers. Methods to such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, proteins, peptides or polypeptides. These derivatives can then be tested for binding according to screening procedures known in the art, e.g. phage display.

The term "antibody" as used herein includes both polyclonal and monoclonal antibodies, as well as any modifications or fragments thereof, such as Fv, Fab and F(ab)2 fragments that are capable of binding antigen or hapten.

In another preferred embodiment, the ligand, preferably chosen from the group consisting of nucleic acids, proteins, peptides, polypeptides, more preferably from the group consisting of nucleic acids, antibodies, or aptamers, is present on an array.

Said array contains at least one additional ligand, which may be directed against a protein, peptide or polypeptide interest. Said additional ligand may also be directed against a protein, peptide, or polypeptide of no particular interest in the context of the present invention. Preferably, ligands for at least three, preferably at least five, more preferably at least eight proteins, peptides or polypeptides of interest in the context of the present invention are contained on the array.

According to the present invention, the term "array" refers to a solid-phase or gel-like carrier upon which at least two compounds are attached or bound in one-, two- or threedimensional arrangement. Such arrays (including "gene chips", "protein chips", antibody arrays and the like) are generally known to the person skilled in the art and typically generated on glass microscope slides, specially coated glass slides such as polycation-, nitrocellulose- or biotin-coated slides, cover slips, and membranes such as, for example, membranes based on nitrocellulose or nylon.

The array may include a bound ligand or at least two cells expressing each at least one ligand.

It is also contemplated to use "suspension arrays" as arrays according to the present invention (Nolan and Sklar, 2002). In such suspension arrays, the carrier, e.g. a microbead or microsphere, is present in suspension. The array consists of different microbeads or microspheres, possibly labeled, carrying different ligands.

The present invention also relates to a kit comprising a means or an agent for measuring MRP 8/14. Optionally, the kit may also comprise means or agents for measuring and markers of myocardial necrosis and/or inflammatory markers and/or metalloproteinases or any other biomarker mentioned in this specification (e.g. myoglobin, creatine kinase, CK-MB, troponin T and troponin I etc.). Such means or agent may be any suitable means or agent known to the person skilled in the art. Examples for such means or agents as well as methods for their use have been given in this specification. For example, a suitable agent may be any kind of ligand or antibody specific for measuring MRP 8/14, or markers of myocardial necrosis and/or inflammatory markers and/or metalloproteinases respectively. The kit may also comprise any other components deemed appropriate in the context of measuring the level(s) of the respective biomarkers, such as suitable buffers, filters, etc.

Optionally, the kit may additionally comprise a user's manual for interpreting the results of any measurement(s) with respect to determining the risk of an individual of suffering from an ACS. Particularly, such manual may include information about what measured level corresponds to what kind of risk group. This is outlined in detail elsewhere in this specification. In an embodiment of the present invention the kit comprises a user's manual disclosing that if an increased level of MRP 8/14 corresponds to a serum level of at least 8.0 mg/L, then the individual is at risk of suffering from an ACS and/or disclosing that if a not elevated level of MRP 8/14 corresponds to a serum level of less than 8.0 mg/L, then the individual does not suffer from an ACS. Additionally, such user's manual may provide instructions about correctly using the components of the kit for measuring the level(s) of the respective biomarkers.

The present invention also relates to the use of said kit for determining a risk whether an individual is suffering from an ACS. The present invention further relates to the use of said kit in any of the methods as mentioned above. A preferred embodiment relates to the use of a kit comprising a means or an agent for measuring MRP 8/14, for determining the risk of an individual of suffering from an ACS. Another preferred embodiment of the present invention relates to the use of a kit comprising a means or an agent for measuring MRP 8/14 to rule out the likelihood whether an individual showing at least one symptom of an evolving ACS is suffering from an ACS. According to the present invention, the measured level of MRP 8/14 indicates whether an individual is suffering from an ACS. The same applies analogously if the levels of MRP 8/14 and markers of myocardial necrosis and/or inflammatory markers and/or metalloproteinases are measured in combination. The terms used in this context, i.e. "non-increased level", "not elevated level", and "increased levels" are known to the person skilled in the art.

For example, MRP 8/14 is usually present in all individuals and can be found in cells, tissues and fluids in all parts of the human body with a biological variation of concentration according to the functional purpose of the protein in the respective tissue. The person skilled in the art is able to determine actual values for the relevant markers, and to define the "non-increased level", "not elevated level", and "increased levels" which usually may be subject to certain variations with respect to the sampling location, the methods of processing, and the technique of the assays applied. For example, the levels may be assigned according to percentiles of the levels observed in a representative sample of apparently healthy individuals below an age of 60 years (preferably, the sample comprises at least 100, more preferably at least 500, most preferably at least 1000 individuals). E.g., a non-increased level may correspond to the maximum level observed in the 97.5% percentile. Alternatively, the levels may be determined as "normal ranges" as known in the state of the art.

The levels may also be determined or further refined by studies performed on individuals showing at least one symptom of an ACS and correlating any cardiovascular event with the levels observed in the individuals. Such studies may also allow to tailor the levels according to certain patient sub-groups, e.g. patients with known coronary artery disease, elderly patients, or individuals merely suffering from chest pain. Guidance on how such studies may be carried out can also be obtained from the Examples included in this specification.

The value of the levels considered as "increased" may also be chosen according to the desired sensitivity or specificity (stringency) of exclusion. The higher the desired sensitivity, the lower is the specificity of exclusion and vice versa. In the above example, the higher the percentile chosen to determine each level, the more stringent is the exclusion criterion, i.e. less individuals would be considered "risk individuals". The method according to the present invention also allows to determine the risk or the likelihood respectively of an individual of suffering from an ACS. According to the present invention, the terms "risk" or "likelihood" relate to the probability of a particular incident, more particularly an ACS. The grade of risk can be non-increased, increased, or highly increased. "Non-increased risk" or "no likelihood" means that there is apparently no risk of suffering from an ACS.

The degree of risk is associated with the levels of MRP 8/14 (or MRP 8/14 and a marker of myocardial necrosis and/or inflammatory marker and/or metalloproteinase. A non-increased level MRP 8/14 indicates no increased risk, an increased level of MRP 8/14 indicates an increased risk, and a highly increased level of MRP 8/14 indicates a highly increased risk. In the case of combined measurement of MRP 8/14 and a marker of myocardial necrosis and/or inflammatory markers and/or metalloproteinases the risks are calculated analogously. However, it may be considered sufficient to indicate a highly increased risk if only one of the markers, i.e. either MRP 8/14 or a marker of myocardial necrosis and/or inflammatory markers and/or metalloproteinases, is highly increased, whereas the level of the respective other marker is merely increased.

As already mentioned, if the level of MRP 8/14 is non-increased, patient does not need urgent intensive coronary care, but may be subjected to further elective, non cardiac or cardiac examinations according to their symptoms. The same applies in the case of combined measurement if the levels of both MRP 8/14 and markers of myocardial necrosis and/or inflammatory markers and/or metalloproteinases are non-increased.

As mentioned above, if the methods according to the present invention indicate an increased or highly increased risk, it will preferably have consequences for the further treatment of the individual.

The present invention is now described in more detail with the following examples or figures to which it is of course not limited.

The present invention is now described in more detail with the following examples or figures to which it is of course not limited.

### Examples

### Example 1:

### Patients and procedures

The study included a total of 116 patients. All patients underwent coronary angiography. The hypothesis was generated from the results of immunohistochemical staining of thrombi harvested from the site of plaque rupture in 41 patients with ACS. The subsequent consecutive 75 study patients were stratified according to clinical presentation, angiographic findings, and the target material assessed.

### Example 2

### Study population for the hypothesis generating analysis of thrombus and plaque material from the site of coronary occlusion

From 41 patients undergoing primary or urgent coronary percutaneous intervention for ST-segment elevation myocardial infarction (STEMI; acute prolonged chest pain and ECG ST-segment elevations >0.2 mV in two contiguous leads) or non-ST-segment elevation myocardial infarction (NSTEMI; typical chest pain at rest with the last episode occurring no longer than 24 hours before admission and an elevated cardiac troponin T level > 0.03 µg/L) respectively, coronary thrombi were removed using a temporary occlusion and aspiration system as previously described (Maier et al., 2005).

### Example 3

### Study population for assessment of inflammatory markers

Patients undergoing coronary angiography at the University Hospital of Zurich were categorized in three groups based on clinical presentation (elective/emergency) and angiographic findings.
A) Normal coronary arteries (n=14): This group consisted of patients without angiographic evidence of coronary artery disease. Coronary artery disease was defined by angiographic documentation of any diameter stenosis of more than 50% in any major epicardial coronary artery. The group included patients undergoing diagnostic coronary angiography prior to valvular surgery and patients with chest pain.
B) Stable coronary artery disease (CAD, n=22): Patients with at least one or more diameter stenosis of more than 50% in any major epicardial coronary artery and angiographic findings consistent with stable CAD. This was considered to be the case, if none of the coronary lesions showed the below listed morphologic signs suggestive of plaque rupture or intra-coronary thrombus formation.
C) Patients with unstable CAD (n=39). This group included patients presenting with an ACS and a clearly identifiable culprit lesion, with at least two of the following five angiographic aspects: a) Ambrose lesions type II (Ambrose et al., 1985a; Ambrose et al., 1985b) b) lesions with exulcerations or irregularities c) signs of thrombus formation (i.e., acute total occlusion, filling defects, haziness in the absence of calcifications, inhomogenous opacification, contrast staining) d) flow impairment in the vessel section distal to the lesion or e) impairment of the wall motion in the corresponding wall segments. ACS comprehended patients with STEMI, NSTEMI and unstable angina (typical chest pain at rest with the last episode occurring no longer than 24 hours before admission and either a documented history of coronary artery disease or ischemic ECG changes defined as ST-segment depression >0.05 mV, transient ST-segment elevation exceeding 0.05 mV, or T-wave inversion >0.2 mV in two contiguous leads). Exclusion criteria were a serum level of C-reactive protein (CRP) ≥ 10 mg/L, inflammatory conditions of the myocardium and pericardium (i.e. myocarditis, pericarditis, transient apical ballooning), a left ventricular ejection fraction of ≤35%, or overt congestive heart failure.

### Example 4

### Subgroup of study population with local and systemic assessment

In 7 of the individuals with unstable CAD, inflammatory markers were assessed both locally at the site of coronary occlusion and systemically.

### Example 5

### Local harvesting of blood, plaque, and thrombus

In the 41 patients with thrombus aspiration and the 7 patients with local and systemic blood samples, the culprit artery was intubated with a 7F guiding catheter and wired with the 0.014 inch PercuSurge™ GuardWire™ (Maier et al., 2005). Briefly, after crossing the culprit lesion, the distal protection balloon was inflated for distal occlusion of the culprit vessel. The Export™ aspiration catheter was inserted over the guide wire, and one passage of aspiration with a syringe from the distal end at the site of the occlusive balloon until the upper border of the culprit lesion ensued to collect blood, thrombus, and plaque material from the site of coronary occlusion (figure 1). Subsequently, PCI was performed.

### Example 6

### Procedures

All patients underwent coronary angiography according to standard techniques. Peripheral blood samples were taken from the arterial sheath immediately after puncture of the femoral artery. Patients without CAD and with stable CAD were managed according to current guidelines (Gibbons et al., 1999; Gibbons et al., 2003) and received either conservative, interventional, or surgical treatment. Patients with unstable CAD underwent percutaneous coronary intervention (PCI).

### Example 7

### Parameters and Biochemical Assays

The following markers were assessed in peripheral blood: MRP 8/14, CK, CK-MB, troponin T, and CRP.

MRP 8/14 was detected in serum using a sandwich ELISA (Bühlmann Laboratories, Schönenbuch, Switzerland) with an inter-assay variation of 7.2% and a detection limit of 3.0 µg/l. The specific monoclonal antibody for the MRP 8/14 heterodimer (mAb 27E10) was used as primary antibody and a polyclonal antibody coupled with horseradish peroxidase was used as secondary antibody. The antibody is specific for the MRP 8/14 heterodimer or higher order complexes and does do not bind MRP-8 or MRP-9 monomers (Hessian and Fisher, 2001; Zwadlo et al., 1986). Albumin, myoglobin, CK, and troponin T were analyzed on a Roche Modular Analytics System using commercial assays from Roche Diagnostics (Rotkreuz, Switzerland) with a maximal inter-assay variation of 2.8%, 4.4%, 1.0%, and 3.7%, respectively. CRP was determined on an Immulite 2000 chemiluminescence analyzer using commercial reagents from Diagnostic Products Corporation (Los Angeles, USA) with a maximal inter-assay variation of 6.3% and a detection limit of 0.1 mg/L.

### Example 8

### Immunohistochemistry

The solid material was kept in buffered paraformaldhyde 4% for 4-6 hours and in 50% ethanol for 24 hours prior to paraffin embedding (Leica EG 1160) and xylol/ethanol fixation (Hypercenter XP, Shandon) and cutting with a microtome (Leica RM2035). The slides were treated with trypsin for 1 h and blocked with 4% fat-free milk in Tris-HCl pH 7.6 for 30 minutes. Monoclonal murine antibodies to human MRP 8/14 (IgG1, clone 27E10, Acris Antibodies GmbH, Hiddenhausen, Germany (Zwadlo et al., 1986) was used at a concentration of 1 µg/ml and incubated with the slides at 4°C for 18 hours. Murine IgG 1 served as negative control. After washing twice, biotinylated AffinityPure F(ab')2 fragment goat anti-mouse IgG (H+L) (Jackson ImmunoResearch Lab., Cambridgeshire, UK) in 5% horse serum and 1% BSA were added to the slide and incubated for 30 minutes at 22-24°C. After washing again, alkaline phosphatase-conjugated streptavidin (Jackson ImmunoResearch Lab.) was added and incubated for 30 minutes at 22-24°C. The staining was developed using the Dako Fast Red Substrate System (DakoCytomation, Glostrup, Denmark).

### Example 9

### Data analysis and statistics

Data are given as mean±standard deviation or as medians [interquartile range, IQR] according to their distribution as assessed by the Kolmogorov-Smirnov test. Levels of inflammatory markers were identified as not normally distributed. Therefore, groups were compared using the Kruskal-Wallis Test and if there were significant differences, the Wilcoxon rank sum test was applied for further pairwise group comparisons. Spearman's rank-correlation test was used to find associations among systemic arterial levels of MRP 8/14 and CRP, as well as other continuous variables such as leukocyte count etc. A correlation coefficient rₛ≥0.7 was considered as good correlation, whereas a rₛ≤0.5 was regarded as a weak association. A two-way cross-tabulation with the chi-square test was used to assess differences in proportions. In order to test the accuracy of MRP 8/14 in detecting a culprit lesion, a receiver-operating-characteristic (ROC) analysis was performed. The ROC-curve was described by the cut-off value (MRP 8/14 serum level with the best sensitivity and specificity) and the area under the curve. Statistical analysis was performed using jmp™ statistical discovery™ (version 6.0; SAS institute, Inc.).

P values < 0.05 were considered as significant.

### Example 10

### Patient characteristics

The characteristics of the 41 consecutive patients with ACS included in the hypothesis-generating immunohistochemical analysis of thrombi are summarized in table 1 (see next page).

**Table 1**

| **ACS patients with immunoshistochemical analysis of thrombi** | |
|---|---|
| **CHARACTERISTIC** | **ACS Patients (n=41)** |
| **Demographics** | |
| Age - yr | 54.1±9.1 |
| Male - no. (%) | 33 (80) |

| **Risk factors** | |
|---|---|
| Hypercholesterolemia - no. (%) | 24 (59) |
| Hypertension - no. (%) | 13 (32) |
| Diabetes mellitus - no. (%) | 6 (15) |
| Family history - no. (%) | 10 (24) |
| Smoking - no. (%) | 29 (71) |

| **Prior medication** | |
|---|---|
| Aspirin - no. (%) | 7 (17) |
| Statin - no. (%) | 7 (17) |

| **Clinical presentation** | |
|---|---|
| Time from index event to angiography - min | 245 [179-323] |

| **ECG on admission** | |
|---|---|
| Normal - no. (%) | 2 (5) |
| Neg. T waves >0.2 mV - no. (%) | 1 (2) |
| ST-depression >0.05 mV - no. (%) | 3 (7) |
| ST-elevation - no. (%) | 35 (86) |

| **Laboratory values on admission** | |
|---|---|
| Leukocytes - x1000/uL | 12.4±4.8 |
| Creatinine - µmol/L | 82.6±14.0 |

| **Angiographic findings** | |
|---|---|
| 1-vessel disease - no. (%) | 23 (56) |
| 2-vessel disease - no. (%) | 12 (29) |
| 3-vessel disease - no. (%) | 6 (15) |
| Chronic Occlusions - no. (%) | 5 (12) |
| Calcifications - no. (%) | 11 (27) |
| LVEF, % | 55.1±11.2 |

| **Culprit lesion** | |
|---|---|
| Culprit vessel - no. (%) | |
| LAD | 25 (61) |
| RCA | 10 (24) |
| LCx | 6(15) |
| Stent thrombosis - no. (%) | 1 (2) |
| Degree of stenosis ≥ 90% - no. (%) | 41 (100) |
| Initial flow < TIMI grade III - no. (%) | 40 (98) |

| **Percutaneous Coronary Intervention** | |
|---|---|
| GP-IIb/IIIa antagonist - no. (%) | 37 (90) |
| PCI time - min | 25 [18-37] |

| **Course** | |
|---|---|
| QwMI - no. (%) | 34 (83) |
| Peak troponin T - µg/L | 4.6 [2.9-6.8] |
| Peak CK-MB - U/L | 302 [134-419] |

### Example 11

The characteristics of the 75 consecutive patients stratified for presentation are summarized in table 2.

**Table 2**

| **Study population for assessment of inflammatory markers** | | | | |
|---|---|---|---|---|
| **CHARACTERISTIC** | **No CAD (n=14)** | **Stable CAD (n=22)** | **Unstable CAD (n=39)** | **p-Value** |
| **Demographics** | | | | |
| Age - yr | 60.4±13.9 | 66.1±12.4 | 58.4±12.0 | ns |
| Male - no. (%) | 10(71) | 17 (77) | 30 (77) | ns |

| **Risk factors** | | | | |
|---|---|---|---|---|
| Hypercholesterolemia - no. (%) | 3(21) | 15 (68) ‡ | 19 (49) | **0.02** |
| Hypertension - no. (%) | 7 (50) | 15 (68) | 15 (38) | ns |
| Diabetes mellitus - no. (%) | 2 (14) | 5 (23) | 3 (8) | ns |
| Family history - no. (%) | 6 (43) | 6 (27) | 9 (23) | ns |
| Smoking - no. (%) | 1 (7) | 7(32) | 19 (49) ‡ | **0.02** |
| PROCAM -Score, % | 4.0 [1.0-17.0] | 8.5 [4.0-16.0] | 7.0 [2.5-13.0] | ns |

| **Prior medication** | | | | |
|---|---|---|---|---|
| Aspirin - no. (%) | 9 (64) | 18 (82) | 5 (13) † | **<0.0001** |
| Betablockers - no. (%) | 8 (57) | 17 (77) | 7 (18) † | **<0.0001** |
| ACEI/ ATB - no. (%) | 6 (43) | 8 (36) | 7 (18) | ns |
| Statin - no. (%) | 6 (43) | 15 (68) | 7 (18) † | **0.0005** |

| **Clinical presentation** | | | | |
|---|---|---|---|---|
| Emergency - no. (%) | 4(29) | 2 (9) | 39(100) † | **<0.0001** |

| **ECG** | | | | |
|---|---|---|---|---|
| Normal - no. (%) | 8 (57) | 7 (31) | 1 (3) † | **<0.0001** |
| ST-elevation - no. (%) | 0 | 0 | 29 (74) † | **<0.0001** |
| Other ST changes - no. (%) | 6 (43) | 15 (68) ¶ | 9 (23) | **0.003** |

| **Laboratory values** | | | | |
|---|---|---|---|---|
| Leukocytes - x1000/uL | 6.9±0.8 | 7.3±1.6 | 10.7±3.6 | **<0.0001** |
| CRP - mg/L | 1.8 [1.1-3.3] | 1.5 [0.9-3.6] | 1.8 [0.8-4.1] | ns |
| Creatinine - µmol/L | 87.6±12.2 | 94.2±15.9 | 87.9±15.9 | ns |

| **Angiographic findings** | | | | |
|---|---|---|---|---|
| 1-vessel disease - no. (%) | 0 | 5 (23) | 20 (51) † | **0.001** |
| 2-vessel disease - no. (%) | 0 | 9 (41) | 10 (26) | **0.02** |
| 3-vessel disease - no. (%) | 0 | 8 (36) | 9 (23) | **0.04** |
| Chronic Occlusions - no. (%) | 0 | 7 (32) † | 3 (8) | **0.008** |
| Calcifications - no. (%) | 0 | 14 (64) † | 7 (18) | **0.0008** |
| LVEF, % | 69.5 [63.0-72.3] | 62.0 [56.0-67.5] | 53.5 [45.8-58.3]† | **<0.0001** |

| | | | | |
|---|---|---|---|---|
| †P<0.05 for the comparison with the other two groups ‡P<0.05 for the comparison with the group with no CAD ¶P<0.05 for the comparison with the group with unstable CAD | | | | |

### Example 12

### Immunohistochemical staining of aspirated material and thrombi from the site of coronary occlusion

Thirty-one (76%) out of 41 consecutive thrombi analyzed revealed positive immunohistochemical stainings for MRP 8/14. The morphology of MRP 8/14+ cells suggested that they belong to the populations of phagocytes. In particular, an infiltrative clustering of MRP 8/14+ cells was observed. Serial sections confirmed that both macrophages (CD68+) and granulocytes (CD66b+) were present in the regions stained for MRP 8/14. Very few lymphocytes have been detected in the thrombi, and no MRPB/14-staining has been seen for lymphocytes (CD3) or thrombocytes (CD42a/b) (data not shown). Systemic and/or local blood samples were not analyzed for MRP 8/14 in this series. Based on these local immunohistochemical findings showing abundant presence of the protein, and the pathophysiological role of MRP 8/14, the hypothesis was generated that this marker might be an indicator of unstable coronary lesions.

### Example 13

### Local versus systemic levels of MRP 8/14 in Patients with ACS

At the site of coronary occlusion, serum levels of MRP 8/14 were increased compared with the systemic circulation (22.0 [16.2-41.5] versus 13.4 [8.1-14.7] mg/L, p < 0.05; figure 2).

### Example 14

### Systemic serum levels of MRP 8/14 in three discriminating clinical conditions: absence of coronary pathology, stable CAD, and acute coronary syndromes

Patients with ACS showed highly increased serum levels of MRP 8/14 compared with both subjects with normal coronary arteries and patients with severe CAD (15.1[12.1-21.8] versus 4.8[4.0-6.3] versus 4.6[3.5-7.1] mg/L, p<0.0001; figure 3). A cut-off value of 8.0 mg/L was determined from ROC analysis which discriminates an acute coronary syndrome with a sensitivity of 95%, a specificity of 92%, and an AOC of 0.97.

In contrast, serum levels of CRP showed no differences between groups (data not shown).

### Example 15

### Correlation of serum levels of MRP 8/14 with examples of other parameters

There was no correlation of MRP 8/14 with CRP levels. A weak correlation of MRP 8/14 with leukocyte counts was observed (rₛ=0.5, p<0.0001). Arterial and venous MRP 8/14 serum levels showed a good correlation (rₛ=0.7, p<0.01).

### Example 16

### Appearance of MRP 8/14 from first symptoms compared with myoglobin, CK-MB, and troponin

In patients presenting with MI, MRP 8/14 was positive in 100% already within 0-3 hours from first symptoms, earlier than any of the classical markers myoglobin, troponin T and CK-MB (figure 5).

### Example 17

### Clinical cases

The following 2 patients (patient A and patient B) presenting with acute chest pain to the University Hospital of Zurich were selected as representative examples, because despite a similar clinical presentation, one patient had an underlying ACS, whereas the other had normal coronary arteries without evidence of CAD. Both patients were female, 56 years old, had a history of hypertension, and presented after two hours of chest pain at rest. The pain was oppressing and located retrosternally in both cases, but could not be provoked by manual pressure on the chest or by forced inspiration. Clinical examination did not reveal any other findings except for patient A who showed a hypertensive blood pressure (204/105 mmHg) in contrast to patient B (118/86). Electrocardiography (ECG) on admission demonstrated ST-segment depressions (>0.05 mV) in leads V2-V5 in both cases. Initial plasma levels of myocardial necrosis, namely myoglobin, CK-MB and troponin T, all were negative in both patients.

From these initial findings an ACS could not be ruled out and, accordingly, the two patients were treated like an ACS. This included continuous ECG-monitoring, and serial assessment of markers of myocardial necrosis. Furthermore patients were given aspirin, heparin, betablockers, and nitrates.

The second blood sample from patient B, drawn 5 hours from admission, showed elevated levels of myoglobin (188 µg/L), CK-MB (70 U/L), and troponin T (1.69 µg/L), clearly indicating an ACS in this patient. In contrast, patient A's second blood analysis was negative again. However, this patient continuously suffered from chest pain and furthermore showed dynamic ST-segment changes, suggestive of ongoing coronary processes. Therefore, both patients underwent coronary angiography, which showed normal coronary arteries in patient A and an ACS with a clearly identifiable culprit lesion (acute occlusion of the circumflex artery) in patient B. In accordance with the present invention, the serum levels of MRP 8/14, taken at angiography, were above the cut-off value (15.1 mg/L) for the patient with ACS (patient B), whereas patient A with no angiographic signs of either unstable or stable CAD, showed an non-elevated MRP 8/14 value (4.3 mg/L).

### Reference list

Alpert et al., J Am Coll Cardiol 2000;36(3):959-69.
Ambrose et al., J Am Coll Cardiol 1985a;5(3):609-16.
Ambrose et al., J Am Coll Cardiol 1985b;6(6):1233-8.
Bhardwaj et al., Eur J Immunol 1992;22(7):1891-7.
Braunwald, Circulation 1989;80(2):410-4.
Burkhardt et al., J Am Soc Nephrol 2001;12(9):1947-57.
Campeau, Circulation 1976;54(3):522-3.
Cesari et al., Circulation 2003;108(19):2317-22.
Cummins et al., Am Heart J 1987;113(6):1333-44.
De Lemos et al., Circulation 2003;107(5):690-5.
De Rycke et al., J Pathol 2005;206(1):17-27.
Eue et al., Int Immunol 2000;12(11):1593-604.
Foell et al., Clin Chim Acta 2004;344(1-2):37-51.
Frosch et al., Arthritis Rheum 2000;43(3):628-37.
Fuster et al., Lancet 1999;353 Suppl 2:SII5-9.
Gibbons et al., Circulation 1999;99(21):2829-48.
Gibbons et al., Circulation 2003;107(1):149-58.
Hamm and Braunwald, Circulation 2000;102(1):118-22.
Hamm et al., N Engl J Med 1997;337(23):1648-53.
Hansson, N Engl J Med 2005;352(16):1685-95.
Healy et al., Circulation 2006; 113(19):2278-84.
Hessian et al., J Leukoc Biol 1993;53(2):197-204.
Hessian and Fisher, Eur J Biochem 2001;268(2):353-63.
Katus et al., J Mol Cell Cardiol 1989;21(12):1349-53.
Liuzzo et al., N Engl J Med 1994;331 (7):417-24.
Liuzzo et al., J Am Coll Cardiol 1999;34(6):1696-703.
Maier et al., Circulation 2005;111(11):1355-61.
Messerli et al., N Engl J Med 2005;353(12):1205-7.
Murao et al., Acta Histochem Cytochem 1994;27:107-16.
Newby et al., Circulation 2001;103(14):1832-7.
Nolan and Sklar, Trends Biotechnol. 2002; 20(1):9-12.
Odink et al., Nature 1987;330(6143):80-2.
Rammes et al., J Biol Chem 1997;272(14):9496-502.
Ridker et al., N Engl J Med 2000;342(12):836-43.
Roth et al., Immunobiology 1992;186(3-4):304-14.
Rugtveit et al., Gut 1994;35(5):669-74.
Schafer et al., Trends Biochem Sci 1996;21(4):134-40.
Stroncek et al., J Transl Med 2005;3:36.
Teigelkamp et al., J Biol Chem 1991;266(20):13462-7.
Widimsky et al., Eur Heart J 2003;24(1):94-104.
Zwadlo et al., J Immunol 1986;137(2):512-8.

WO 2004/057341

## Claims

1. A method for determining the risk whether an individual showing at least one symptom of an evolving acute coronary syndrome is suffering from an acute coronary syndrome, comprising the steps of
a. measuring, preferably in vitro, the level of MRP 8/14 or a variant thereof, wherein
b. if the level of MRP 8/14 or a variant thereof is at least increased, then the individual is at risk of suffering from an acute coronary syndrome.

2. The method according to claim 1, which comprises the further step of
aa. measuring, preferably in vitro, the level of markers of myocardial necrosis and/or inflammatory markers and/or metalloproteinases or a variant thereof,
and the modified step
ba. if the levels of both the markers of myocardial necrosis and/or inflammatory markers and/or metalloproteinases and MRP 8/14, or a respective variant thereof, are at least increased, then the individual is at risk of suffering from an acute coronary syndrome.

3. The method according to any of claims 1 or 2, wherein the individual is showing chest pain and/or breathlessness.

4. The method according to any of claims 1 to 3, wherein the level of MRP 8/14 is measured in serum and/or plasma.

5. The method according to any of claims 1 to 4, wherein an increased level of MRP 8/14 corresponds to a serum level of at least 8.0 mg/L.

6. The method according to any of claims 1 to 5, wherein additionally the level(s) of one or more of the following biomarkers are measured:
a) myoglobin,
b) creatine kinase,
c) creatine kinase-MB,
d) troponin T,
e) troponin I

7. A method to rule out the likelihood whether an individual showing at least one symptom of an evolving acute coronary syndrome is suffering from an acute coronary syndrome, comprising the steps of
a. measuring, preferably in vitro, the level of MRP 8/14 or a variant thereof, wherein
b. if the level of MRP 8/14 or a variant thereof is not elevated, then the individual does not suffer from an acute coronary syndrome.

8. A method to assess whether an individual showing at least one symptom of an evolving acute coronary syndrome is not at risk of suffering from an acute coronary syndrome, comprising the steps of
a. measuring, preferably in vitro, the level of MRP 8/14 or a variant thereof, wherein
b. if the level of MRP 8/14 or a variant thereof is not elevated, then the individual is not at risk of suffering from an acute coronary syndrome.

9. A method to discriminate if an individual showing at least one symptom of an evolving acute coronary syndrome is at risk to suffer from an acute coronary syndrome or has no acute coronary syndrome, comprising the steps of
a. measuring, preferably in vitro, the level of MRP 8/14 or a variant thereof, wherein
b. if the level of MRP 8/14 or a variant thereof is at least increased, then the individual is at risk of suffering from an acute coronary syndrome,
c. if the level of MRP 8/14 or a variant thereof is not elevated, then the individual has no acute coronary syndrome.

10. The method according to any of claims 7 to 9, wherein a not elevated level of MRP 8/14 corresponds to a serum level of less than 8.0 mg/L.

11. Use of MRP 8/14 or a variant thereof for determining the risk whether an individual showing at least one symptom of an evolving acute coronary syndrome is suffering from an acute coronary syndrome, comprising the steps of
a. measuring, preferably in vitro, the level of MRP 8/14 or a variant thereof, wherein
b. if the level of MRP 8/14 or a variant thereof is at least increased, then the individual is at risk of suffering from an acute coronary syndrome.

12. A kit comprising a means or an agent for measuring MRP 8/14, wherein the kit comprises a user's manual for interpreting the results of any measurement(s) with respect to determining the risk of an individual of suffering from an acute coronary syndrome.

13. A kit according to claim 12, wherein the user's manual discloses that if an increased level of MRP 8/14 corresponds to a serum level of at least 8.0 mg/L, then the individual is at risk of suffering from an acute coronary syndrome.

14. Use of a kit comprising a means or an agent for measuring MRP 8/14, for determining the risk of an individual of suffering from an acute coronary syndrome.

15. Use of a kit comprising a means or an agent for measuring MRP 8/14, in a method according to any of claims 1 to 10.
